(19) Europäisches Patentamt / European Patent Office / Office européen des brevets

(11) **EP 3 590 510 B1**

(12) **EUROPEAN PATENT SPECIFICATION**

(45) Date of publication and mention of the grant of the patent:
**04.05.2022 Bulletin 2022/18**

(21) Application number: **18761811.1**

(22) Date of filing: **02.03.2018**

(51) International Patent Classification (IPC):
**A61K 9/14** $^{(2006.01)}$    **A61K 31/12** $^{(2006.01)}$
**G01N 21/3563** $^{(2014.01)}$    **G01N 33/15** $^{(2006.01)}$

(52) Cooperative Patent Classification (CPC):
**A61K 9/146; A61K 31/12; A61P 3/04; A61P 3/06;**
**A61P 3/10; A61P 9/12; A61P 37/08;**
**G01N 21/3563; G01N 33/15**

(86) International application number:
**PCT/JP2018/008187**

(87) International publication number:
**WO 2018/159853 (07.09.2018 Gazette 2018/36)**

(54) **CURCUMIN-CONTAINING MEDICINAL PREPARATION AND METHOD FOR EVALUATING ABSORPTION OR ELUTION CHARACTERISTICS THEREOF**

CURCUMINHALTIGE MEDIZINISCHE ZUBEREITUNG UND VERFAHREN ZUR BEURTEILUNG VON ABSORPTIONS- ODER ELUTIONSEIGENSCHAFTEN DAVON

PRÉPARATION MÉDICINALE CONTENANT DE LA CURCUMINE ET PROCÉDÉ D'ÉVALUATION DES CARACTÉRISTIQUES D'ABSORPTION OU D'ÉLUTION DE CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.03.2017 JP 2017041174**

(43) Date of publication of application:
**08.01.2020 Bulletin 2020/02**

(73) Proprietors:
• **San-Ei Gen F.F.I., INC.**
  **Toyonaka-shi, Osaka 561-8588 (JP)**
• **Osaka University**
  **Suita-shi, Osaka 565-0871 (JP)**

(72) Inventors:
• **NAGANO, Kazuya**
  **Suita-shi**
  **Osaka 565-0871 (JP)**
• **HIGASHISAKA, Kazuma**
  **Suita-shi**
  **Osaka 565-0871 (JP)**
• **TSUTSUMI, Yasuo**
  **Suita-shi**
  **Osaka 565-0871 (JP)**
• **KINOSHITA, Keigo**
  **Toyonaka-shi**
  **Osaka 561-8588 (JP)**

(74) Representative: **Vossius & Partner**
**Patentanwälte Rechtsanwälte mbB**
**Siebertstrasse 3**
**81675 München (DE)**

(56) References cited:
**WO-A1-2006/083001    JP-A- H10 509 796**
**US-A1- 2010 256 748**

• **RAHMA A. ET AL.: "Intermolecular interactions and the release pattern of electrospun curcumin-polyvinyl(pyrrolidone) fiber", BIOLOGICAL & PHARMACEUTICAL BULLETIN, vol. 39, no. 2, 1 January 2016 (2016-01-01), pages 163-173, XP055543804, JP ISSN: 0918-6158, DOI: 10.1248/bpb.b15-00391**
• **RADJARAM A. ET AL.: "Dissolution enhancement of curcumin by hydroxypropy 1-b-cyclodextrin complexation", INT.J.PHARM.PHARM.SCI., vol. 5, no. suppl.3, 1 January 2013 (2013-01-01), XP055543797, England ISSN: 0075-1491**

- KAKRAN M. ET AL.: "Ternary dispersions to enhance solubility of poorly water soluble antioxidants", COLLOIDS AND SURFACES A: PHYSIOCHEMICAL AND ENGINEERING ASPECTS, ELSEVIER, AMSTERDAM, NL, vol. 433, 13 May 2013 (2013-05-13), pages 111-121, XP028570665, ISSN: 0927-7757, DOI: 10.1016/J.COLSURFA.2013.05.021
- RADJARAM, A. et al.: "Dissolution enhancement of curcumin by hydroxypropy 1-b-cyclodextrin complexation", Int.J.Pharm.Pharm.Sci., vol. 5, no. Suppl.3, 2013, pages 401-405, XP055543797,
- KAKRAN, M. et al.: "Ternary dispersions to enhance solubility of poorly water soluble antioxidants", Colloids and Surfaces A:Physicochem. Eng. Aspects, vol. 433 20 September 2013 (2013-09-20), pages 111-121, XP028570665, Retrieved from the Internet: URL:https://doi.org/10.1016/j.colsurfa.2013.05.021
- RAHMA, A. et al.: "Intermolecular interactions and the release pattern of electrospun curcumin-polyvinyl (pyrrolidone) fiber", Biol. Pharm. Bull., vol. 39, no. 2 2016, pages 163-173, XP055543804, Retrieved from the Internet: URL:doi: 10.1248/bpb.b15-00391
- SADEGHI, F. et al.: "Anti sol vent precipitation technique :A very promising approach to crystallize curcumin in presence of polyvinyl pyrrolidon for solubility and dissolution enhancement", Colloids and Surfaces B: Biointerfaces, vol. 147 1 November 2016 (2016-11-01), pages 258-264, XP029734138, Retrieved from the Internet: URL:https://doi.org/10.1016/j.colsurfb.2016.08.004
- WANG, C. et al.: "Enhanced bioavailabili ty and anticancer effect of curcumin- loaded electrospun nano fiber: In vitro and in vivo study", Nanoscale Research Letters, vol. 10, no. 439 2015, pages 1-10, XP055543806, Retrieved from the Internet: URL:doi: 10.1186/s11671-015-1146-2

Remarks:

The file contains technical information submitted after the application was filed and not included in this specification

**Description**

Technical Field

The present invention relates to a curcumin-containing preparation.
The present invention further relates to a method for evaluating absorption or dissolution ability etc. of the preparation.

Background Art

Curcumins are believed to have physiological effects, such as suppression of cholesterol elevation, suppression of blood pressure elevation, suppression of blood glucose elevation, antiallergic effects, suppression of body fat, and the like.
To expect such physiological effects, the ingestion of large amounts of curcumin is necessary.
Curcumins are components contained in, for example, edible plants. Although curcumins can be ingested, for example, in usual meals, ingesting curcumins in the form of curcumin-containing tablets or like solid composition is convenient and efficient.
However, most curcumins are poorly soluble in water. Therefore, even if a curcumin-containing solid composition is ingested, curcumins are dissolved and absorbed into the body fluid at slow rates.
To solve this problem, for example, Patent Document 1 suggests an oral composition comprising a curcuminoid and an essential oil of turmeric.
However, from the aspects of efficient ingestion of curcumin, development of furthermore novel technique is required.

Citation List

Patent Documents

Patent Document 1: JP2012-188450A

Summary of Invention

Technical Problem

In view of the background art described above, an object of the present invention is to provide a curcumin-containing preparation that enables efficient ingestion of curcumin.
Further, to efficiently develop such an excellent curcumin-containing preparation, it is useful to provide (1) a simple method for predicting curcumin-dissolubility into body fluid, and (2) a simple method for predicting curcumin-absorbability of a curcumin-containing preparation into a body.
Therefore, another object of the present invention is to provide these methods, as well as media, devices, and the like for use in the methods.

Solution to Problem

The inventors of the present invention carried out extensive research and found that the above problem can be solved by:

[A] A curcumin-containing preparation, comprising amorphous curcumin and being substantially free of crystalline curcumin, wherein, in an infrared absorption spectrum with curve fitting by a Voigt function, a ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$ is 0.25 or less;
[B] a method for predicting curcumin-dissolubility of the curcumin-containing preparation into body fluid, comprising:

(1) a stage of performing infrared spectroscopic analysis of a curcumin-containing preparation;
(2) a stage of performing curve fitting with respect to an infrared absorption spectrum obtained in stage (1) using a Voigt function;
(3) in the curve-fitted infrared absorption spectrum obtained in stage (2), a stage of calculating the ratio (Cp/Ap) of peak intensity Cp having the maximum in the range of 1508.00 to 1513.00 $cm^{-1}$ to peak intensity Ap having the maximum in the range of 1513.50 to 1517.00 cm$^{-1}$; and

(4) a stage of assuming high curcumin-dissolubility into body fluid when the ratio (Cp/Ap) is small; and

[C] a method for predicting curcumin-absorbability of the curcumin-containing preparation into a body, the method comprising:

(1) a stage of performing infrared spectroscopic analysis of a curcumin-containing preparation;
(2) a stage of performing curve fitting with respect to an infrared absorption spectrum obtained in stage (1) using a Voigt function;
(3) in the curve-fitted infrared absorption spectrum obtained in stage (2), a stage of calculating the ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and
(4) a stage of assuming high curcumin-absorbability into a body when the ratio (Cp/Ap) is small.

With this finding, the inventors completed the present invention.
The present invention encompasses the following aspects.

[Item 1.]

A curcumin-containing preparation comprising amorphous curcumin and being substantially free of crystalline curcumin, wherein, in an infrared absorption spectrum with curve fitting by a Voigt function, a ratio (Cp/Ap) is 0.25 or less, the ratio (Cp/Ap) being a ratio of peak intensity Cp having the maximum in the range of 1508.00 to 1513.00 cm$^{-1}$ to peak intensity Ap having the maximum in the range of 1513.50 to 1517.00 cm$^{-1}$.

[Item 2.]

The preparation according to Item 1, wherein the content of curcumin is 10 mass% or more based on the entire preparation.

[Item 3.]

A method for predicting curcumin-dissolubility of a curcumin-containing preparation into body fluid, the method comprising:

(1) a stage of performing infrared spectroscopic analysis of a curcumin-containing preparation;
(2) a stage of performing curve fitting with respect to an infrared absorption spectrum obtained in stage (1) using a Voigt function;
(3) in the curve-fitted infrared absorption spectrum obtained in stage (2), a stage of calculating a ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and
(4) a stage of assuming high curcumin-dissolubility into body fluid when the ratio (Cp/Ap) is small.

[Item 4.]

A method for predicting curcumin-absorbability of a curcumin-containing preparation into a body, the method comprising:

(1) a stage of performing infrared spectroscopic analysis of a curcumin-containing preparation,
(2) a stage of performing curve fitting with respect to an infrared absorption spectrum obtained in stage (1) using a Voigt function,
(3) in the curve-fitted infrared absorption spectrum obtained in stage (2), a stage of calculating a ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and
(4) a stage of assuming high curcumin-absorbability into a body when the ratio (Cp/Ap) is small.

[Item 5.]

A computer-readable medium storing computer code, the computer code functioning to

(1) perform curve fitting with respect to an infrared absorption spectrum obtained by infrared spectroscopic analysis of a curcumin-containing preparation using a Voigt function;

(2) calculate a ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and

(3) based on the ratio (Cp/Ap), output

    (a) an estimate of curcumin-dissolubility into body fluid, and/or
    (b) an estimate of curcumin-absorbability into a body.

[Item 6.]

A device for predicting curcumin-dissolubility of a curcumin-containing preparation, the device comprising:

(1) an analysis section for performing curve fitting with respect to an infrared absorption spectrum obtained by infrared spectroscopic analysis of a curcumin-containing preparation using a Voigt function,

(2) a calculation section for calculating a ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and

(3) an output section for outputting, based on the ratio (Cp/Ap),

    (a) an estimate of curcumin-dissolubility into body fluid, and/or
    (b) an estimate of curcumin-absorbability into a body.

[Item 7.]

The device according to Item 6, wherein the device causes a computer to function as the analysis section (1), the calculation section (2), and the output section (3).

Advantageous Effects of Invention

The present invention provides a curcumin-containing preparation that enables efficient ingestion of curcumin.
The present invention further provides (1) a simple method for predicting curcumin-dissolubility into body fluid, and (2) a simple method for predicting curcumin-absorbability of the curcumin-containing preparation into a body, so as to efficiently develop such an excellent curcumin-containing preparation.

Brief Description of Drawings

Fig. 1 is an IR chart of the preparation of Comparative Example B1.
Fig. 2 is an IR chart of the preparation of Example B2.
Fig. 3 illustrates one exemplary configuration of the prediction device of the present invention.

Description of Embodiments

Term

The symbols and abbreviations used in this specification can be assumed to have their ordinary meanings used in the technical field to which the present invention pertains, as understood from the context of the specification, unless otherwise specified.
In the specification, the terms "containing" and "comprising" are used to include meanings of the phrase "consisting essentially of" and the phrase "consisting of."
The step, treatment, or operation disclosed in the specification can be performed at room temperature, unless otherwise specified. In this specification, room temperature refers to a temperature in the range of 10 to 40°C.

1. Curcumin-containing Preparation

The curcumin-containing preparation of the present invention comprises amorphous curcumin and is substantially free of crystalline curcumin.

In an infrared absorption spectrum with curve fitting by a Voigt function, the ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$ is 0.25 or less.

[1-1] Curcumin

The curcumin-containing preparation of the present invention comprises amorphous curcumin and is substantially free of crystalline curcumin.

For example, curcumin can be obtained as an extract of a natural product, or from commercial suppliers.

The lower limit of curcumin content in the preparation of the present invention is, for example, 5 mass%, preferably 7 mass%, more preferably 10 mass%, further preferably 15 mass%, and further more preferably 20 mass%.

The upper limit of curcumin content in the preparation of the present invention is, for example, 60 mass%, 50 mass%, 45 mass%, 40 mass%, 35 mass%, or 30 mass%.

The curcumin content in the preparation of the present invention is, for example, in the range of 5 to 60 mass%, preferably in the range of 7 to 50 mass%, more preferably in the range of 10 to 45 mass%, further preferably in the range of 15 to 40 mass%, and further more preferably in the range of 20 to 35 mass%.

The preparation of the present invention comprises amorphous curcumin and is substantially free of crystalline curcumin. In this specification, "free of" may mean "essentially free of," "substantially free of," or "completely free of."

The proportion of crystalline curcumin in the preparation of the present invention may specifically be less than 10 mass%, preferably less than 5 mass%, more preferably less than 3 mass%, and further preferably less than 1 mass%.

The curcumin-containing preparation of the present invention comprises amorphous curcumin and is substantially free of crystalline curcumin, and this formulation can be confirmed by a known method such as powder X-ray diffraction or differential scanning calorimetry.

Similarly, the content of the crystalline curcumin relative to the entire curcumin in the preparation can be determined by powder X-ray diffraction, differential scanning calorimetry, or like methods.

If the powder X-ray diffraction finds that the content of crystalline curcumin is not more than the analytical limit, the actual content of crystalline curcumin may be assumed to be less than 3 mass% at most. This means that crystalline curcumin is not substantially contained.

Further, if the differential scanning calorimetry finds that the content of crystalline curcumin is not more than the analytical limit, the actual content of crystalline curcumin may be assumed to be less than 1 mass% at most. This means that crystalline curcumin is not substantially contained.

In the curcumin-containing preparation of the present invention, in an infrared absorption spectrum with curve fitting by a Voigt function, the ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$ is 0.25 or less.

As is generally known, the Voigt function is expressed by the following formula:

$$y = y_0 + A\frac{2\ln 2}{\pi^{3/2}}\frac{W_L}{W^2{}_G}\int_{-\infty}^{\infty}\frac{e^{-t^2}}{\left(\sqrt{\ln 2}\frac{W_L}{W_G}\right)^2 + \left(\sqrt{4\ln 2}\frac{x - x_c}{W_G} - t\right)^2}dt$$

The symbols in the formula and the infrared absorption spectrum parameters of the present invention have the following relationship.

x: wavenumber
y: transmittance or absorbance
A: peak area
$x_c$: peak position
$W_G$ (weighting coefficient of Gaussian function): peak width
$W_L$ (weighting coefficient of Lorenz function): peak width

Further, the ratio (Cp/Ap) is preferably 0.25 or less, more preferably 0.20 or less, further preferably 0.15 or less, and further more preferably 0.14 or less.

When the ratio has the value specified above, the curcumin-containing preparation of the present invention ensures high curcumin-dissolubility into body fluid.

An infrared absorption spectrum with curve fitting by a Voigt function may be obtained by a method comprising the following stages.

Stage (1A): infrared absorption spectrum analysis is performed under the following conditions.

- ▪ Conditions for Infrared Absorption Spectrum Analysis
- ▪ An infrared spectrometer (PerkinElmer, Frontier IR) or a similar product
- • Potassium bromide (KBr) disk method

Stage (2A): data having information of a wavenumber ($cm^{-1}$) as the X-axis and a transmittance (%T) as the Y-axis obtained by an infrared spectrometric measurement is obtained.
Stage (2B): waveform is analyzed using waveform analysis conditions and software having a waveform analysis function (OriginPro 2017J b9.4.0.220, or a similar product).

Whether an appropriate waveform analysis was performed can be confirmed by confirming that the coefficient of determination $R^2$ of the approximate curve obtained by the waveform analysis is more than 0.997 (preferably 0.999), and that there is no peak having a negative peak height between the range of 1508.00 to 1517.00 $cm^{-1}$.

[1-2] Components other than curcumin

The curcumin-containing preparation of the present invention may comprise components other than curcumin.
The components other than curcumin include general components for use in a preparation (e.g., orally administered preparation).
Examples of the components other than curcumin include hydrophilic polymers and nonionic surfactants.
Preferable examples of the curcumin-containing preparation of the present invention include a preparation comprising:

(1) curcumin;
(2) hydrophilic polymer; and
(3) at least one nonionic surfactant selected from the group consisting of polyglycerol fatty acid esters, sucrose fatty acid esters, and lecithins.

(1-2-1) Hydrophilic polymer

The hydrophilic polymer used in the present invention is not necessarily hydrophilic or water-soluble under every condition. The hydrophilic polymer is preferably hydrophilic or water-soluble at least at the pH in the intestinal tract.
The hydrophilic polymer used in the present invention is preferably a solid at room temperature.
The hydrophilic polymer used in the present invention preferably has a glass transition temperature (Tg) of preferably about 50°C or more, more preferably about 80°C to about 180°C. The determination of the glass transition temperature (Tg) can be performed according to JIS K 7121: 2012.
The preparation of the present invention may contain one hydrophilic polymer, or two or more hydrophilic polymers.
Examples of the hydrophilic polymer used in the present specification include the followings.

(1) homopolymers of N-vinyllactam (preferably N-vinylpyrrolidone) (e.g., polyvinylpyrrolidones (i.e., PVP or povidone) (e.g., Kollidon™ 12PF, Kollidon™ 17PF, Kollidon™ 25, Kollidon™ 30, Kollidon™ 90F, or equivalents thereof), and copolymers thereof (e.g., a copolymer of N-vinylpyrrolidone and vinyl acetate monomers (i.e., copovidone), or a copolymer of N-vinylpyrrolidone and vinyl propionate monomers);
(2) cellulose esters and cellulose ethers, in particular, methyl cellulose, ethyl cellulose, hydroxyalkyl cellulose (e.g., hydroxypropyl cellulose (i.e., HPC)), hydroxyalkyl alkyl cellulose (e.g., hydroxypropyl methylcellulose (namely, HPMC)), or hypromellose (e.g., Methocel™ E3, Methocel™ E5, Methocel™ E6, Methocel™ E15, or equivalents thereof, Methocel™ K3, or equivalents thereof), cellulose phthalate, and cellulose succinate (e.g., cellulose acetate phthalate, hydroxypropyl methylcellulose phthalate, hydroxypropyl methylcellulose succinate, and hydroxypropyl methylcellulose acetate succinate (i.e., HPMC-AS));
(3) high-molecular-weight polyalkylene oxides (e.g., polyethylene oxide, polypropylene oxide, and copolymers of ethylene oxide and propylene oxide (e.g., poloxamers));
(4) polyacrylate and polymethacrylate (e.g., methacrylic acid/ethyl acrylate copolymer, methacrylic acid/methyl methacrylate copolymer, butyl methacrylate/2-dimethylaminoethyl methacrylate copolymer, poly(hydroxyalkyl acrylate), and poly(hydroxyalkyl methacrylate));

(5) polyacrylamides;
(6) vinyl acetate polymers and copolymers of polyvinyl alcohol; and oligosaccharide and polysaccharides (e.g., carrageenan, galactomannan, and xanthan gum);
and mixtures of two or more of the above compounds.

In one preferable embodiment of the present invention, the preparation of the present invention may contain as the hydrophilic polymer at least one member selected from the group consisting of polyvinylpyrrolidone, hydroxypropyl cellulose, and hydroxypropyl methylcellulose, and may further contain other hydrophilic polymers.

In one particularly preferable embodiment of the present invention, the preparation of the present invention may contain at least a polyvinylpyrrolidone as the hydrophilic polymer and may further contain other hydrophilic polymers.

In another preferable embodiment of the present invention, the hydrophilic polymer is at least one member selected from the group consisting of polyvinylpyrrolidone, hydroxypropyl cellulose, and hydroxypropyl methylcellulose.

In another particularly preferable embodiment, the hydrophilic polymer is polyvinylpyrrolidone.

The hydrophilic polymer content is preferably within the range of 5 to 90 mass%, more preferably within the range of 20 to 90 mass%, and even more preferably within the range of 40 to 90 mass%.

(1-2-2) Nonionic surfactant

The nonionic surfactant is a nonionic surfactant that is at least one member selected from the group consisting of polyglycerol fatty acid esters, sucrose fatty acid esters, and lecithins.

Examples of polyglycerol fatty acid esters used in the present invention include esters of (a) polyglycerols having an average degree of polymerization of 2 or more (preferably 3 to 15, more preferably 3 to 10), and (b) fatty acids having 8 to 18 carbon atoms (e.g., caprylic acid, capric acid, lauric acid, myristic acid, palmitic acid, stearic acid, oleic acid, and linoleic acid).

Specific examples of polyglycerol fatty acid esters used in the present invention include diglycerol monolaurate, diglycerol monostearate, diglycerol monooleate, decaglycerol monolaurate, decaglycerol monostearate, and decaglycerol monooleate.

In the present invention, the polyglycerol fatty acid esters can be used singly or in a combination of two more.

The HLB value of sucrose fatty acid esters used in the present invention is preferably 5 or more, more preferably 7 or more, further preferably 10 or more, and further more preferably 12 or more.

The fatty acid of the sucrose fatty acid ester used in the present invention preferably has at least 12 carbon atoms, and more preferably 12 to 20 carbon atoms.

Specific examples of sucrose fatty acid esters preferably used in the present invention include sucrose laurate, sucrose myristate, sucrose palmitate, sucrose stearate, sucrose oleate, sucrose behenate, and sucrose erucate.

In the present invention, the sucrose fatty acid esters can be used singly or in a combination of two or more.

The lecithin used in the present invention is an adduct of a phosphoric acid derivative of di-fatty acid ester of glycerol (diglyceride). Lecithin is widely distributed in plant and animal bodies.

Examples of the lecithin used in the present invention include egg yolk lecithin contained in egg yolk, soybean lecithin contained in soybeans, and sunflower lecithin contained in sunflowers.

Examples of the lecithin used in the present invention include fractionated lecithin obtained by extracting an active ingredient from a lecithin described above, enzymatically modified lecithin obtained by treating lecithin with an enzyme, and enzymatically decomposed lecithin.

Specific examples of the lecithin used in the present invention include lecithin, enzymatically decomposed lecithin (phosphatidic acid), lysolecithin, soybean lecithin (soybean phospholipid), and egg yolk lecithin.

Lecithins that can be used in the present invention are commercially available. For example, SLP-White (trade name, produced by Tsuji Oil Mill Co., Ltd.) can be used.

In the present invention, lecithins can be used singly, or in a combination of two or more.

Particularly suitable examples of the nonionic surfactant contained in the preparation of the present invention include polyglycerol fatty acid esters.

The preparation of the present invention may contain one or more nonionic surfactants.

In a preferred embodiment of the present invention, the nonionic surfactant is a polyglycerol fatty acid ester.

The nonionic surfactant content in the preparation of the present invention is preferably within the range of 5 to 90 mass%, more preferably within the range of 5 to 60 mass%, and further preferably within the range of 10 to 40 mass%.

(1-3) Other components

If necessary, the preparation of the present invention may further contain components other than those mentioned above, as long as the effects of the present invention are not significantly impaired.

Examples of such components include excipients, fillers, extenders, binders, disintegrators, surfactants, seasonings, flavoring agents, and lubricants.

As long as the effect of the present invention is not significantly impaired, the types and amounts of such components may be suitably selected and designed based on common general technical knowledge.

[1-4] Method for producing curcumin-containing preparation

The curcumin-containing preparation of the present invention (which may also be referred to as a "preparation of the present invention" in this specification) may be produced by a production method comprising a step of mixing, for example,

(1) crystalline curcumin;
(2) hydrophilic polymer; and
(3) at least one nonionic surfactant selected from the group consisting of polyglycerol fatty acid esters, sucrose fatty acid esters, and lecithins; and
(4) other optional components.

The method comprises the step of converting the crystalline curcumin to amorphous curcumin.

In the mixing step, the above components can be mixed simultaneously or successively.

The mixing step can be preferably performed without using a solvent such as an organic solvent.

Even when a solvent is used, the above components, such as curcumin, do not have to be completely dissolved in the solvent.

This allows the preparation of the present invention to be produced at low cost without using a large container or the like.

The step of mixing the components and the step of converting the crystalline curcumin to amorphous curcumin can be separate steps, or they can be partially or completely in common.

A higher conversion of crystalline curcumin to amorphous curcumin is preferable. Converting all or substantially all the crystalline curcumin to amorphous curcumin is particularly preferable.

The preparation of the present invention can be produced, for example, by solvent precipitation methods, spray-drying methods, freeze-drying methods, drying under reduced pressure, or kneading methods, or a combination of these methods.

The preparation of the present invention is preferably produced by a production method comprising the step of kneading:

(1) crystalline curcumin;
(2) hydrophilic polymer;
(3) at least one nonionic surfactant selected from the group consisting of polyglycerol fatty acid esters, sucrose fatty acid esters, and lecithins; and
(4) other optional components.

In the kneading step, the crystalline curcumin, the hydrophilic polymer, and the nonionic surfactant are preferably kneaded simultaneously.

The kneading converts a part of the crystalline curcumin to amorphous curcumin, or preferably converts all or substantially all the crystalline curcumin to amorphous curcumin.

The kneading can be preferably performed, for example, by using a single-screw extruder, an intermeshing screw extruder, or a multi-screw extruder (e.g., a twin-screw extruder). The kneading can also be preferably performed by kneading with a relatively weak force, such as kneading by hand using a spatula or the like on a hot plate. In this kneading, for example, the mixture is kneaded while heated to the temperature at which the components are dissolved; then, after the components are dissolved, the mixture is cooled to room temperature. The resulting preparation is pulverized into a powder using a pulverizer to obtain the preparation of the present invention.

Primary-particle diameter of the preparation of the present invention may be, preferably, for example, within the range of 0.1 to 500 $\mu$m. The particle diameter can be measured by dynamic light scattering (DLS).

The preparation of the present invention is preferably produced by, for example, a method comprising the steps of: fully mixing the crystalline curcumin, the hydrophilic polymer, and the nonionic surfactant with an oil or fat to prepare a slurry in which the curcumin is dissolved; and drying the slurry.

Examples of drying methods include spray-drying methods, freeze-drying methods, vacuum-drying methods, drum-drying methods, far-infrared drying methods, and the like. Spray-drying methods are particularly preferable.

[2] Method for Predicting Curcumin-dissolubility of the curcumin-containing preparation into Body Fluid

The method for predicting ability of the curcumin-containing preparation of the present invention to dissolve curcumin into body fluid comprises the following stages (1) to (4) .

Stage (1) performs infrared spectroscopic analysis of a curcumin-containing preparation.

Stage (1) may be performed, for example, by the method of stage (1A) explained in regard to the method for obtaining an infrared absorption spectrum with curve fitting by a Voigt function in the description of the curcumin-containing preparation of the present invention.

Stage (2) performs curve fitting with respect to the infrared absorption spectrum obtained in stage (1) using a Voigt function.

Stage (2) may be performed, for example, by the methods of stages (2A) and (2B) explained in regard to the method for obtaining an infrared absorption spectrum with curve fitting by a Voigt function in the description of the curcumin-containing preparation of the present invention.

Stage (3) performs, in the curve-fitted infrared absorption spectrum obtained in stage (2), calculation of the ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$.

Stage (3) may be performed in accordance with common technical knowledge of a person skilled in the art; usually, the intensity may be read as the peak intensity of the peak defined herein in the curve-fitted infrared absorption spectrum obtained in stage (2).

In stage (4), the curcumin-dissolubility of the curcumin-containing preparation into body fluid is assumed to be high when the ratio (Cp/Ap) is small.

More specifically, in stage (4), the curcumin-dissolubility into body fluid is determined or predicted to be higher when the ratio (Cp/Ap) is smaller, and the curcumin-dissolubility into body fluid is assumed to be lower when the ratio (Cp/Ap) is greater.

In view of assumption accuracy, the ratio (Cp/Ap) is preferably in a range of 0 to 5, more preferably in a range of 0 to 1, and further preferably in a range of 0 to 0.5.

In this specification, examples of body fluids include blood, gastric fluid, intestinal fluid, extracellular fluid and intracellular fluid of mammals (e.g., humans).

In the present invention, for example, if the value of Cp/Ap is 0.25 or less, it can be predicted that the AUC (area under the blood concentration-time curve) obtained by the method in the absorption test (blood curcumin concentration) described later in the Examples is 10000 or more. Further, in the present invention, for example, if the value of Cp/Ap is 0.15 or less, it can be predicted that the AUC (area under the blood concentration-time curve) obtained by the method in the absorption test (blood curcumin concentration) described later in the Examples is 15000 or more.

Regarding this matter, for the sake of precaution, it should be noted that the act of predicting that the AUC (area under the blood concentration-time curve) value B obtained by the method in the absorption test (blood curcumin concentration) described later in the Examples is equal to or more than a predetermined value (e.g., 10000) when the Cp/Ap value A is equal to or less than, for example, 0.25, encompasses the act of predicting a AUC value (e.g., 15000) greater than the value B by the value A' (e.g., 0.15) smaller than the value A of the Cp/Ap.

The "method for predicting curcumin-dissolubility of the curcumin-containing preparation into body fluid" of the present invention enables simple prediction of curcumin-dissolubility.

Therefore, it is possible to greatly improve the efficiency of tests of newly developed preparations, the efficiency of quality evaluation of actually produced preparations, and the like.

[3] Method for Predicting Ability of the Curcumin-Containing Preparation to Enable Curcumin to Be Absorbed Into a Body

The method for predicting ability of the curcumin-containing preparation of the present invention to enable curcumin absorption into a body comprises the following stages (1) to (4) .

Stage (1) performs infrared spectroscopic analysis of a curcumin-containing preparation.

Stage (2) performs curve fitting with respect to the infrared absorption spectrum obtained in stage (1) using a Voigt function.

Stage (3) performs, in the curve-fitted infrared absorption spectrum obtained in stage (2), calculation of the ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$.

These stages (1) to (3) may respectively be the same as stages (1) to (3) explained above in the "Method for Predicting Curcumin-dissolubility of the curcumin-containing preparation into Body Fluid" section.

In stage (4), the curcumin-absorbability of the curcumin-containing preparation into a body is assumed to be high when the ratio (Cp/Ap) is small.

More specifically, in stage (4), the curcumin-absorbability into a body is determined or predicted to be higher when the ratio (Cp/Ap) is smaller, and the curcumin-absorbability into a body is assumed to be lower when the ratio (Cp/Ap) is greater.

In the present invention, "curcumin absorption into a body" may mean curcumin absorption into cells (e.g., into the cytoplasm or cell membrane), tissues, organs, and/or organ systems.

In view of assumption accuracy, the ratio (Cp/Ap) is preferably in a range of 0 to 5, more preferably in a range of 0 to 1, and further preferably in a range of 0 to 0.5.

The method for predicting curcumin-absorbability of the curcumin-containing preparation into a body of the present invention also enables simple prediction of curcumin-dissolubility.

Therefore, it is possible to greatly improve the efficiency of tests of newly developed preparations, the efficiency of quality evaluation of actually produced preparations, and the like.

[4] A Computer-Readable Medium Storing Computer Code

Further, the present invention also provides a computer-readable medium storing computer code, wherein the computer code functions to:

(1) perform curve fitting with respect to an infrared absorption spectrum obtained by infrared spectroscopic analysis of a curcumin-containing preparation using a Voigt function;
(2) calculate a ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and
(3) based on the ratio (Cp/Ap), output

(a) an estimate of the curcumin-dissolubility into body fluid, and/or
(b) an estimate of the curcumin-absorbability into a body.

This medium can be understood from the descriptions of this specification and common technical knowledge.

[5] Prediction Device

Further, the present invention also provides a device for predicting a curcumin-dissolubility of a curcumin-containing preparation, the device comprising:

(1) an analysis unit for performing curve fitting with respect to an infrared absorption spectrum obtained by infrared spectroscopic analysis of a curcumin-containing preparation using a Voigt function;
(2) a calculation section for calculating a ratio (Cp/Ap) of peak intensity Cp having the maximum in the range of 1508.00 to 1513.00 cm$^{-1}$ to peak intensity Ap having the maximum in the range of 1513.50 to 1517.00 cm$^{-1}$; and
(3) an output section for outputting, based on the ratio (Cp/Ap),

(a) an estimate of the curcumin-dissolubility into body fluid, and/or
(b) an estimate of the curcumin-absorbability into a body.

This device can be understood from the descriptions of this specification and common technical knowledge.

This device enables a computer to appropriately function as the analysis section (1), the calculation section (2), and the output section (3).

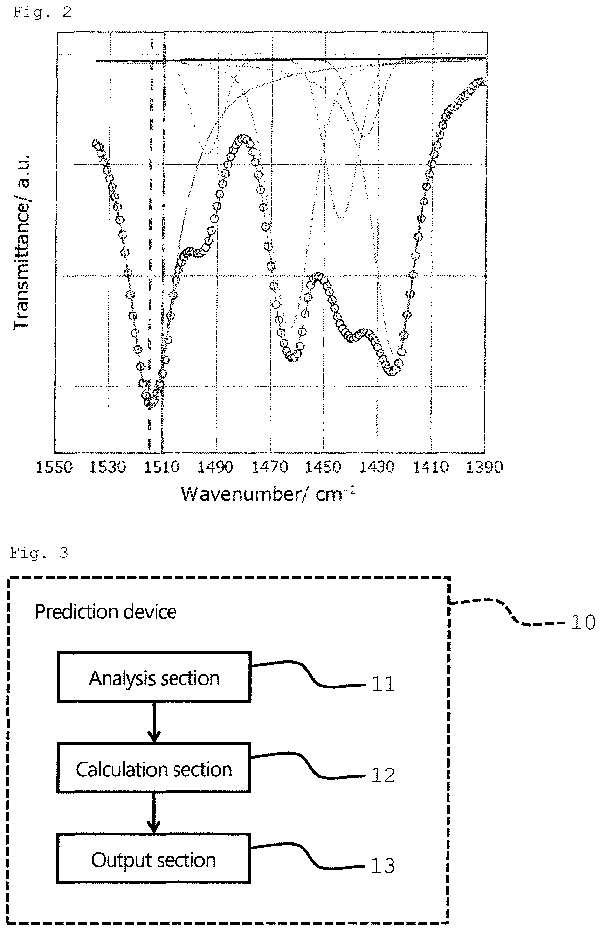

Fig. 3 shows an example of the structure of the prediction device.

In a prediction device 10, curve fitting is performed by an analysis section 11 with respect to an infrared absorption spectrum obtained by infrared spectroscopic analysis of a curcumin-containing preparation using a Voigt function.

The ratio (Cp/Ap) is calculated from the curve-fitted waveform by a calculation section 12.

Based on the ratio (Cp/Ap), the calculation section 12 further calculates:

(a) an estimate of the curcumin-dissolubility into body fluid, and/or
(b) an estimate of the curcumin-absorbability into a body.

The estimate may be, but is not particularly limited to, the form of a specific numerical value or a rank.

The estimate is outputted from an output section 13.

Examples

The present invention is described in more detail below with reference to Examples. However, the scope of the present invention is not limited to these Examples.

The symbols and abbreviations in the Examples are defined as follows.

CUR: curcumin
PVP: polyvinylpyrrolidone
PGFE: polyglycerin fatty acid ester
NIS: nonionic surfactant
SE: sucrose fatty acid ester (sugar ester)

Method for Preparing Samples

While compositions having the formulations shown in Table 1 below were heated on a hot plate to their melting temperatures, the compositions were kneaded by hand using a spatula. After melting, each melted product was cooled to room temperature, and pulverized into a powder using a pulverizer. Each powder thus obtained was used as a sample.

Kneading with heating was performed by setting the hot plate to 240°C, and kneading each composition on the plate by hand using a spatula or the like until the composition was melted.

The components used in the Examples or Comparative Examples are described below (the symbols described in this paragraph may be used hereinafter to indicate these components).

Components

CUR (curcumin): curcumin material (purity: 90 mass% or more curcumin) (bulk powder)
PVP (polyvinylpyrrolidone): Kollidon K30 (trade name, BASF)
PGFE: Ryoto Polyglyester M-10D (trade name, Mitsubishi Chemical Foods Co., Ltd.)
SE-1: Ryoto Sugar Ester S-1570 (trade name, Mitsubishi Chemical Foods, Inc.) (stearic acid ester, HLB15)
SE-2: Ryoto Sugar Ester S-770 (trade name, Mitsubishi Chemical Foods, Inc.) (stearic acid ester, HLB7)

Table 1 shows the formulations of the preparations.

Here, as can be understood from the formulations of the preparations, all of the preparations of Comparative Example B1, Example B1, Example B2, and Example B3 have a constant CUR/PVP ratio of 30/70, whereas the amount of PGFE is different within the range of 0% to 35%.

In the table, a peak Cp intensity of 0.000 means that no peak Cp was observed, as is generally understood.

Infrared Absorption Analysis

Each preparation was subjected to infrared absorption analysis. The infrared absorption analysis was performed under the following conditions:

Conditions

- Infrared spectroscopy device (Perkin Elmer, Inc., Frontier IR).
- Potassium bromide (KBr) tablet method
- Waveform separation by Voigt function was performed by inputting wavenumber ($cm^{-1}$) and transmittance (%T) data (wavenumber range: 1390 to 1535 $cm^{-1}$) into OriginPro 207 J b9. 4.1.220. The straight line connecting the end points was used as the baseline. The coefficient of determination for the approximate curve $R^2$ being greater than 0.999, and no peaks with a negative peak height within the range of 1513.50 to 1517.00 $cm^{-1}$, confirmed that the waveform analysis was properly performed.

Table 1 shows the infrared absorption analysis results.

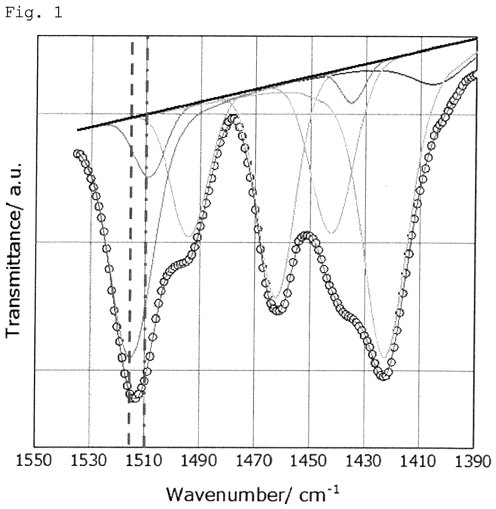

Figs. 1 and 2 show IR charts of the preparation of Comparative Example B1 and the preparation of Example B2.

In each graph, a horizontal solid line or a diagonal solid line indicates the baseline. The vertical dashed line (---) represents the wavenumber of 1515 cm$^{-1}$. The vertical dash-dot line (-·-·) represents the wavenumber of 1510 cm$^{-1}$.

In the graphs, white circles (○) are plots of the measured values. The line almost perfectly overlapping the white circles is a waveform synthesized from the waveforms obtained by curve-fitting using the Voigt function. The lines not overlapping the white circles are waveforms obtained by curve-fitting using the Voigt function.

Absorption Test (Blood Curcumin Concentration)

The changes over time of blood curcumin concentration in rats to which the above preparations were administered were examined by the following test methods. As a comparative example, curcumin bulk powder was administered.

Testing Method

Animals: Three SD rats (male, 7 weeks old, fasted for 14 to 16 hours before administration) per group were used.
Administration: 100 mg/Kg in terms of curcumin, single oral administration (sonde method).
Blood sampling: Jugular venous blood sampling immediately before administration; and at 0.5, 1, 2, 4, 8, and 24 hours after administration.
Analysis: 25 μl of plasma was enzymatically treated with β-glucuronidase. After curcumin was extracted with acetonitrile, the solvent was evaporated to dryness. The resulting product was diluted again with methanol, and measured by UV detection (420 nm). Table 1 shows the AUC (area under the blood concentration-time curve).

Table 1

| Preparation | CUR (%) | PVP (%) | NIS | NIS (%) | AUC | $R^2$ | Peak Ap Intensity | Peak Ap Position [cm$^{-1}$] | Peak Cp Intensity | Peak Cp Position [cm$^{-1}$] | Cp/Ap Intensity ratio |
|---|---|---|---|---|---|---|---|---|---|---|---|
| Example A1 | 21.0 | 64.0 | PGFE | 15.0 | 19731 | 0.9993 | 8272 | 1514.19 | 0.000 | - | 0.000 |
| Example A2 | 19.0 | 56.0 | PGFE | 25.0 | 27432 | 0.9990 | 6.795 | 1513.92 | 0.000 | - | 0.000 |
| Example A3 | 16.0 | 49.0 | PGFE | 35.0 | 30345 | 0.9992 | 8.981 | 1514.65 | 0.000 | - | 0.000 |
| Comparative Example B1 | 30.0 | 70.0 | - | 0.0 | 5004 | 0.9994 | 9.339 | 1515.00 | 2.476 | 1508.60 | 0.265 |
| Example B1 | 25.5 | 59.5 | PGFE | 15.0 | 12727 | 0.9991 | 13.427 | 1514.97 | 3.124 | 1512.83 | 0.233 |
| Example B2 | 22.5 | 52.5 | PGFE | 25.0 | 20449 | 0.9992 | 15.338 | 1514.04 | 0.000 | - | 0.000 |
| Example B3 | 19.5 | 45.5 | PGFE | 35.0 | 20043 | 0.9990 | 9.253 | 1514.18 | 0.000 | - | 0.000 |
| Example C1 | 22.5 | 52.5 | SE-1 | 25.0 | 21660 | 0.9989 | 24.003. | 1514.78 | 0.000 | - | 0.000 |
| Example C2 | 22.5 | 52.5 | SE-2 | 25.0 | 23025 | 0.9973 | 10.087 | 1514.36 | 0.000 | - | 0.000 |

The results confirmed that the curcumin contained in the preparations of the present invention is highly soluble in blood, and highly absorbable.

**Claims**

1. A curcumin-containing preparation comprising amorphous curcumin and being substantially free of crystalline curcumin, wherein, in an infrared absorption spectrum with curve fitting by a Voigt function, a ratio (Cp/Ap) is 0.25 or less, the ratio (Cp/Ap) being a ratio of peak intensity Cp having the maximum in the range of 1508.00 to 1513.00 $cm^{-1}$ to peak intensity Ap having the maximum in the range of 1513.50 to 1517.00 $cm^{-1}$.

2. The preparation according to claim 1, wherein the content of curcumin is 10 mass% or more based on the entire preparation.

3. A method for predicting curcumin-dissolubility of a curcumin-containing preparation into body fluid, the method comprising:

   (1) a stage of performing infrared spectroscopic analysis of a curcumin-containing preparation;
   (2) a stage of performing curve fitting with respect to an infrared absorption spectrum obtained in stage (1) using a Voigt function;
   (3) in the curve-fitted infrared absorption spectrum obtained in stage (2), a stage of calculating a ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$; and
   (4) a stage of assuming high curcumin-dissolubility into body fluid when the ratio (Cp/Ap) is small.

4. A method for predicting curcumin-absorbability of a curcumin-containing preparation into a body, the method comprising:

   (1) a stage of performing infrared spectroscopic analysis of a curcumin-containing preparation,
   (2) a stage of performing curve fitting with respect to an infrared absorption spectrum obtained in stage (1) using a Voigt function,
   (3) in the curve-fitted infrared absorption spectrum obtained in stage (2), a stage of calculating a ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$; and
   (4) a stage of assuming high curcumin-absorbability into a body when the ratio (Cp/Ap) is small.

5. A computer-readable medium storing computer code, the computer code functioning to

   (1) perform curve fitting with respect to an infrared absorption spectrum obtained by infrared spectroscopic analysis of a curcumin-containing preparation using a Voigt function;
   (2) calculate a ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$; and
   (3) based on the ratio (Cp/Ap), calculate

      (a) an estimate of curcumin-dissolubility into body fluid, and/or
      (b) an estimate of curcumin-absorbability into a body.

6. A device for predicting curcumin-dissolubility of a curcumin-containing preparation, the device comprising:

   (1) an analysis section for performing curve fitting with respect to an infrared absorption spectrum obtained by infrared spectroscopic analysis of a curcumin-containing preparation using a Voigt function,
   (2) a calculation section for calculating a ratio (Cp/Ap) of peak intensity Cp having the maximum in the range of 1508.00 to 1513.00 $cm^{-1}$ to peak intensity Ap having the maximum in the range of 1513.50 to 1517.00 $cm^{-1}$; and
   (3) an output section for outputting, based on the ratio (Cp/Ap),

      (a) an estimate of curcumin-dissolubility into body fluid, and/or
      (b) an estimate of curcumin-absorbability into a body.

7. The device according to claim 6, wherein the device causes a computer to function as the analysis section (1), the calculation section (2), and the output section (3).

**Patentansprüche**

1. Eine curcuminhaltige Zubereitung, welche amorphes Curcumin umfasst und im Wesentlichen frei von kristallinem Curcumin ist, wobei in einem Infrarot-Absorptionsspektrum mit Kurvenanpassung durch eine Voigt-Funktion ein Verhältnis (Cp/Ap) 0,25 oder weniger beträgt, wobei das Verhältnis (Cp/Ap) ein Verhältnis der Peak-Intensität Cp mit dem Maximum im Bereich von 1508,00 bis 1513,00 cm$^{-1}$ zur Peak-Intensität Ap mit dem Maximum im Bereich von 1513,50 bis 1517,00 cm$^{-1}$ ist.

2. Die Zubereitung gemäß Anspruch 1, wobei der Gehalt an Curcumin 10 Massen-% oder mehr, bezogen auf die gesamte Zubereitung, beträgt.

3. Ein Verfahren zur Vorhersage der Curcumin-Löslichkeit einer curcuminhaltigen Zusammensetzung in Körperflüssigkeit, wobei das Verfahren umfasst:

   (1) eine Stufe des Durchführens einer Infrarot-Spektroskopie-Analyse einer curcuminhaltigen Zubereitung;
   (2) eine Stufe des Durchführens einer Kurvenanpassung in Bezug auf ein Infrarot-Absorptionsspektrum, welches in Stufe (1) erhalten wurde, unter Verwendung einer Voigt-Funktion;
   (3) in dem in Stufe (2) erhaltenen kurvenangepassten Infrarot-Absorptionsspektrum, eine Stufe des Berechnens eines Verhältnisses (Cp/Ap) der Peak-Intensität Cp mit dem Maximum im Bereich von 1508,00 bis 1513,00 cm$^{-1}$ zur Peak-Intensität Ap mit dem Maximum im Bereich von 1513,50 bis 1517,00 cm$^{-1}$; und
   (4) eine Stufe der Annahme einer hohen Curcumin-Löslichkeit in Körperflüssigkeit, wenn das Verhältnis (Cp/Ap) klein ist.

4. Ein Verfahren zur Vorhersage der Curcumin-Absorptionsfähigkeit einer curcuminhaltigen Zusammensetzung in einem Körper,
   wobei das Verfahren umfasst:

   (1) eine Stufe des Durchführens einer Infrarot-Spektroskopie-Analyse einer curcuminhaltigen Zubereitung,
   (2) eine Stufe des Durchführens einer Kurvenanpassung in Bezug auf ein Infrarot-Absorptionsspektrum, welches in Stufe (1) erhalten wurde, unter Verwendung einer Voigt-Funktion,
   (3) in dem in Stufe (2) erhaltenen kurvenangepassten Infrarot-Absorptionsspektrum, eine Stufe des Berechnens eines Verhältnisses (Cp/Ap) der Peak-Intensität Cp mit dem Maximum im Bereich von 1508,00 bis 1513,00 cm$^{-1}$ zur Peak-Intensität Ap mit dem Maximum im Bereich von 1513,50 bis 1517,00 cm$^{-1}$; und
   (4) eine Stufe der Annahme einer hohen Curcumin-Absorptionsfähigkeit in einem Körper, wenn das Verhältnis (Cp/Ap) klein ist.

5. Ein computerlesbares Medium, welches einen Computercode speichert,
   wobei der Computercode funktioniert, um

   (1) eine Kurvenanpassung in Bezug auf ein Infrarot-Absorptionsspektrum durchzuführen, welches durch Infrarot-Spektroskopie-Analyse einer curcuminhaltigen Zusammensetzung unter Verwendung einer Voigt-Funktion erhalten wurde;
   (2) ein Verhältnis (Cp/Ap) der Peak-Intensität Cp mit dem Maximum im Bereich von 1508,00 bis 1513,00 cm$^{-1}$ zur Peak-Intensität Ap mit dem Maximum im Bereich von 1513,50 bis 1517,00 cm$^{-1}$ zu berechnen; und
   (3) bezogen auf das Verhältnis (Cp/Ap)

      (a) eine geschätzte Curcumin-Löslichkeit in Körperflüssigkeit und/oder
      (b) eine geschätzte Curcumin-Absorptionsfähigkeit in einem Körper zu berechnen.

6. Eine Vorrichtung zur Vorhersage der Curcumin-Löslichkeit einer curcuminhaltigen Zusammensetzung, wobei die Vorrichtung umfasst:

   (1) einen Analysebereich zur Durchführung einer Kurvenanpassung in Bezug auf ein Infrarot-Absorptionsspektrum, welches durch Infrarot-Spektroskopie-Analyse einer curcuminhaltigen Zusammensetzung unter Verwendung einer Voigt-Funktion erhalten wurde,

(2) einen Berechnungsbereich zur Berechnung eines Verhältnisses (Cp/Ap) der Peak-Intensität Cp mit dem Maximum im Bereich von 1508,00 bis 1513,00 cm$^{-1}$ zur Peak-Intensität Ap mit dem Maximum im Bereich von 1513,50 bis 1517,00 cm$^{-1}$; und

(3) einen Ausgabebereich zum Ausgeben von, bezogen auf das Verhältnis (Cp/Ap),

(a) einer geschätzten Curcumin-Löslichkeit in Körperflüssigkeit und/oder
(b) einer geschätzten Curcumin-Absorptionsfähigkeit in einem Körper.

7. Die Vorrichtung gemäß Anspruch 6, wobei die Vorrichtung einen Computer dazu veranlasst, als der Analysebereich (1), der Berechnungsbereich (2) und als Ausgabebereich (3) zu arbeiten.


**Revendications**

1. Préparation contenant de la curcumine comprenant de la curcumine amorphe et étant pratiquement exempte de curcumine cristalline, dans laquelle, dans un spectre d'absorption infrarouge avec un ajustement de courbe au moyen d'une fonction de Voigt, un rapport (Cp/Ap) est de 0,25 ou moins, le rapport (Cp/Ap) étant un rapport de l'intensité de pic Cp dont le maximum est situé dans la plage allant de 1508,00 à 1513,00 cm$^{-1}$ à l'intensité de pic Ap dont le maximum est situé dans la plage allant de 1513,50 à 1517,00 cm$^{-1}$.

2. Préparation selon la revendication 1, dans laquelle la teneur en curcumine est de 10 % en masse ou plus par rapport à la préparation totale.

3. Procédé pour prédire la capacité de dissolution dans un fluide corporel de la curcumine d'une préparation contenant de la curcumine, le procédé comprenant :

(1) une étape de réalisation d'une analyse spectroscopique infrarouge d'une préparation contenant de la curcumine ;
(2) une étape de réalisation d'un ajustement de courbe en regard d'un spectre d'absorption infrarouge obtenu dans l'étape (1) par utilisation d'une fonction de Voigt ;
(3) dans le spectre d'absorption infrarouge ayant subi un ajustement de courbe obtenu dans l'étape (2), une étape de calcul d'un rapport (Cp/Ap) de l'intensité de pic Cp dont le maximum est situé dans la plage allant de 1508,00 à 1513,00 cm$^{-1}$ à l'intensité de pic Ap dont le maximum est situé dans la plage allant de 1513,50 à 1517,00 cm$^{-1}$ ; et
(4) une étape de supposition qu'une capacité de dissolution de la curcumine dans un fluide corporel est élevée quand le rapport (Cp/Ap) est petit.

4. Procédé pour prédire la capacité d'absorption dans un corps de la curcumine d'une préparation contenant de la curcumine, le procédé comprenant :

(1) une étape de réalisation d'une analyse spectroscopique infrarouge d'une préparation contenant de la curcumine,
(2) une étape de réalisation d'un ajustement de courbe en regard du spectre d'absorption infrarouge obtenu dans l'étape (1) par utilisation d'une fonction de Voigt,
(3) dans le spectre d'absorption infrarouge ayant subi un ajustement de courbe obtenu dans l'étape (2), une étape de calcul d'un rapport (Cp/Ap) de l'intensité de pic Cp dont le maximum est situé dans la plage allant de 1508,00 à 1513,00 cm$^{-1}$ à l'intensité de pic Ap dont le maximum est situé dans la plage allant de 1513,50 à 1517,00 cm$^{-1}$ ; et
(4) une étape de supposition qu'une capacité d'absorption de la curcumine dans un corps est élevée quand le rapport (Cp/Ap) est petit.

5. Support lisible par ordinateur stockant un code informatique, le code informatique fonctionnant de manière à

(1) réaliser un ajustement de courbe en regard d'un spectre d'absorption infrarouge obtenu par analyse spectroscopique infrarouge d'une préparation contenant de la curcumine par utilisation d'une fonction de Voigt ;
(2) calculer un rapport (Cp/Ap) de l'intensité de pic Cp dont le maximum est situé dans la plage allant de 1508,00 à 1513,00 cm$^{-1}$ à l'intensité de pic Ap dont le maximum est situé dans la plage allant de 1513,50 à 1517,00 cm$^{-1}$ ; et

(3) sur la base du rapport (Cp/Ap), calculer

    (a) une estimation d'une capacité de dissolution de la curcumine dans un fluide corporel, et/ou
    (b) une estimation d'une capacité d'absorption de la curcumine dans un corps.

6. Dispositif pour prédire une capacité de dissolubilité de la curcumine d'une préparation contenant de la curcumine, le dispositif comprenant :

    (1) une section d'analyse pour réaliser un ajustement de courbe en regard d'un spectre d'absorption infrarouge obtenu par analyse spectroscopique infrarouge d'une préparation contenant de la curcumine par utilisation d'une fonction de Voigt,
    (2) une section de calcul pour calculer un rapport (Cp/Ap) de l'intensité de pic Cp dont le maximum est situé dans la plage allant de 1508,00 à 1513,00 cm$^{-1}$ à l'intensité de pic Ap dont le maximum est situé dans la plage allant de 1513,50 à 1517,00 cm$^{-1}$ ; et
    (3) une section de sortie pour délivrer en sortie, sur la base du rapport (Cp/Ap),

        (a) une estimation de la capacité de dissolution de la curcumine dans un fluide corporel, et/ou
        (b) une estimation de capacité d'absorption de la curcumine dans un corps.

7. Dispositif selon la revendication 6, lequel le dispositif amène un ordinateur à fonctionner comme la section d'analyse (1), la section de calcul (2), et la section de sortie (3).

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012188450 A